## Europäisches Patentamt

## European Patent Office

## Office européen des brevets

(11) Veröffentlichungsnummer: **0 009 741**
**B1**

(12) **EUROPÄISCHE PATENTSCHRIFT**

(45) Veröffentlichungstag der Patentschrift:
**20.01.82**

(21) Anmeldenummer: **79103584.3**

(22) Anmeldetag: **24.09.79**

(51) Int. Cl.³: **C 07 C 69/16,** C 07 C 69/28,
C 07 C 67/055

(54) Verfahren zur Herstellung von Butendioldiestern.

(30) Priorität: **28.09.78 DE 2842178**

(43) Veröffentlichungstag der Anmeldung:
**16.04.80 Patentblatt 80/8**

(45) Bekanntmachung des Hinweises auf die Patenterteilung:
**20.01.82 Patentblatt 82/3**

(84) Benannte Vertragsstaaten:
**BE DE FR GB NL**

(56) Entgegenhaltungen:

**GB-A-1 003 396**
**US-A-3 850 980**

(73) Patentinhaber: **BASF Aktiengesellschaft,
Carl-Bosch-Strasse 38, D-6700 Ludwigshafen (DE)**

(72) Erfinder: **Weitz, Hans-Martin, Dr. Dipl.-Chem., Auf dem
Koeppel 40, D-6702 Bad Duerkheim 1 (DE)**
Erfinder: **Schwarz, Helmut, Dr. Dipl.-Chem.,
Weinbrennerstrasse 5, D-6700 Ludwigshafen (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

## Verfahren zur Herstellung von Butendioldiestern

Die Erfindung betrifft ein Verfahren zur Herstellung von Butendiol-diacetaten oder -dipropionaten durch Umsetzung von Butadien mit Sauerstoff und Essigsäure bzw. Propionsäure in Gegenwart eines hochsiedenden Lösungsmittels an einem festen Katalysator, der ein Platinmetall und mindestens ein Element der 5. oder 6. Hauptgruppe enthält.

Es ist – z.B. aus der DE-OS 22 17 452 – bekannt, Carbonsäure-Diester eines ungesättigten Diols durch Umsetzung eines konjugierten Diens mit Sauerstoff und einer Carbonsäure an einem festen Katalysator herzustellen, der Palladium und mindestens eines der Elemente Antimon, Wismut, Tellur und Selen enthält. Nach der DE-OS 24 17 658 gelingt die Umsetzung von Butadien mit Sauerstoff und Essigsäure zu Butendioldiacetaten auch an einem festen Katalysator, der Platin und mindestens ein Element der 5. oder 6. Hauptgruppe enthält.

Die im Falle der Verwendung von Essigsäure als Carbonsäure entstehenden Butendioldiacetate (BEDA), sind das cis- bzw. trans-2-Buten-1,4-dioldiacetat (1,4-BEDA) und 1-Buten-3,4-dioldiacetat (3,4-BEDA). Sie haben – bei atmosphärischem Druck – Siedepunkte von 227 bzw. 235 und 206 °C.

Die Siedepunkte der entsprechenden Butendioldipropionate liegen – bei Normaldruck – ca. 20 °C höher.

Da die vorliegende Erfindung vorzugsweise ein Verfahren zur Herstellung von Butendioldiacetaten (BEDA), insbesondere von cis- bzw. trans-2-Buten-1,4-dioldiacetat (1,4-BEDA) und 1-Buten-3,4-dioldiacetat (3,4-BEDA) durch Umsetzung von Butadien mit Sauerstoff und Essigsäure in Gegenwart hochsiedender Lösungsmittel an einem festen Katalysator betrifft, soll im folgenden die Erfindung im einzelnen an diesem Beispiel veranschaulicht werden. Entsprechendes gilt bei Einsatz von Propionsäure anstelle von Essigsäure.

Bei der technischen Durchführung dieser als Acetoxilierungsreaktion bezeichneten Umsetzung nach bisher bekannten Verfahren sind einige Randbedingungen zu berücksichtigen, die die Wirtschaftlichkeit des technischen Verfahrens begrenzen. Hierzu gehört vor allem die Löslichkeit von Sauerstoff in Essigsäure und die erforderliche Wärmeabfuhr von etwa 196 kJ/mol BEDA (bei Einsatz von flüssigem oder in Essigsäure gelöstem Butadien). Zu berücksichtigen sind ferner Polymerenbildung, Einfluss des Wassers, das bei der Reaktion entsteht, Reaktionstemperatur, Katalysatorleistung usw. Aufgrund dieser Voraussetzungen enthält das erzielbare Reaktionsgemisch im allgemeinen neben etwa 1% Wasser nur 5 bis 10% BEDA und im übrigen Essigsäure. Wegen des hohen Siedepunkts der Butendioldiacetate können diese aus dem Reaktionsgemisch nur dadurch isoliert werden, dass neben dem stöchiometrisch anfallenden Wasser grosse Mengen Essigsäure abdestilliert werden. Ausserdem ist es erforderlich, die mit Wasser verdünnte Essigsäure zu entwässern, da beim fortlaufenden Verfahren die Essigsäure wieder in die Synthese zurückgeführt werden muss und dabei nur gewisse Wassermengen toleriert werden können. Schliesslich muss auch das abgetrennte Wasser möglichst essigsäurefrei gemacht werden. Da bekanntlich die Destillation zu den energieaufwendigsten Verfahrensmassnahmen gehört, ist die Wirtschaftlichkeit des gesamten Verfahrens in Frage gestellt. Die Erfindung hat sich die Aufgabe gestellt, ein Verfahren aufzuzeigen, das den Energiebedarf gegenüber den bekannten Verfahren deutlich herabsetzt.

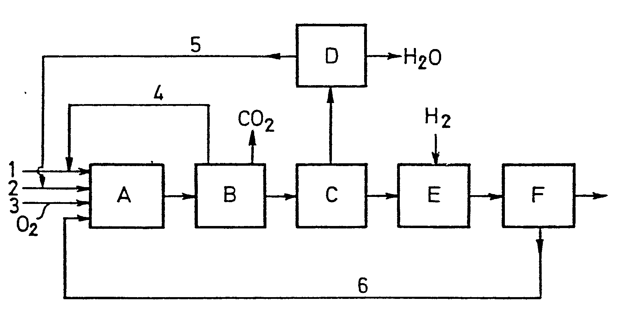

Es wurde gefunden, dass man Butendioldiacetat (BEDA), speziell 1,4-BEDA und 3,4-BEDA, oder Butendioldipropionat kostengünstig herstellen kann durch Umsetzung von Butadien mit Sauerstoff und Essigsäure bzw. Propionsäure oder einer Verbindung, die unter den Reaktionsbedingungen Essigsäure bzw. Propionsäure freisetzt, insbesondere Methylacetat bzw. Methylpropionat an einem festen Katalysator, der ein Platinmetall und mindestens ein Element der 5. und 6. Hauptgruppe enthält, wenn man die Umsetzung in Gegenwart eines Lösungsmittels durchführt, das einen höheren Siedepunkt als die Butendioldiacetate bzw. Butendioldipropionate aufweist und das die Reaktion weder chemisch beeinflusst noch von ihr verändert wird. Dieses Lösungsmittel sollte mit Essigsäure möglichst mischbar sein. Beispiele für solche Lösungsmittel sind Sulfolan (Tetrahydrothiophen-1,1-dioxid) bzw. dessen Substitutionsprodukte (z.B. 3-Methyltetrahydrothiophen-1,1-dioxid), Siliconöle (z.B. Methylphenylpolysiloxane) oder Phthalsäurediester (speziell Di-n-butylphthalat bzw. Dimethoxyäthylphthalat). Geeignete andere Lösungsmittel sind z.B. folgenden Literaturstellen zu entnehmen: Chem. Ztg. 95 (1971) 586 (Tab. 1) und Verfahrenstechnik 7 (1973) 297 (Tab. 4 und Abb. 5).

Bei den bekannten Verfahren zur Herstellung von Butendioldiacetaten wird die Reaktion in Essigsäure durchgeführt, wobei der Essigsäure eine Doppelfunktion als Reaktionspartner und als Lösungsmittel (für Butadien und Sauerstoff) bzw. als Verdünnungsmittel zukommt. Sie wird deshalb in sehr grossem Überschuss eingesetzt und verursacht damit aber auch die bereits erwähnten hohen Energiekosten bei der Aufarbeitung derartiger Reaktionsausträge.

Die Acetoxilierungsreaktion kann im Prinzip mit der stöchiometrisch aus dem eingesetzten Butadien sich ergebenden Menge Essigsäure (2 mol Essigsäure pro mol Butadien) durchgeführt werden. Ein Überschuss an Essigsäure ist natürlich möglich. Im allgemeinen wird man das Verfahren sogar mit einem gewissen Überschuss an Essigsäure betreiben.

Hierbei hat sich folgendes ergeben:

Verringert man die Essigsäuremenge unter Beibehaltung der sonstigen Bedingungen, so ergibt sich zwar eine geringere Reaktionsgeschwindigkeit, jedoch ist die Reaktionsgeschwindigkeit in einem grösseren, besonders interessierenden Mischungsbereich wesentlich, d.h. überproportional höher als dem Anteil der Essigsäure im Gemisch entspricht. Ersetzt man nämlich in einer Versuchsreihe einen Teil der ursprünglich vorhandenen Essigsäure systematisch durch eine entsprechende Menge von z.B. Sulfolan, so sieht man, dass die Reaktionsgeschwindigkeit der Essigsäurekonzentration nicht proportional ist. Dies ist umso überraschender, als es normalerweise Ziel der Verfahrensentwicklung ist, ohne zusätzliche Lösungsmittel auszukommen. Inwieweit man bei einer technischen Durchführung der Acetoxilierung die Essigsäure durch eines der erfindungsgemässen Lösungsmittel ersetzt und wie man das Verhältnis Essigsäure/Butadien wählt, ist durch einen Vorversuch zu ermitteln und hängt auch von wirtschaftlichen Erwägungen ab. Prinzipiell ist jedes Verhältnis möglich. Im allgemeinen wird man pro mol eingesetztes Butadien 0,5 bis 15, insbesondere 3 bis 9 mol Essigsäure einsetzen. Im Lösungsmittelgemisch (Essigsäure/hochsiedende Verbindung) wird der Anteil der hochsiedenden Komponente im allgemeinen zwischen 5 und 80%, insbesondere zwischen 20 und 70% liegen. (Angaben jeweils in Massen-%, bezogen auf Gesamtgemisch.)

Nach dem erfindungsgemässen Verfahren werden nach der Acetoxilierung zunächst nicht umgesetzte oder entstandene gasförmige abzuscheidende Verbindungen entfernt (Butadien, Butene, $CO_2$, ggf. $O_2$); dann entfernt man vorhandenes Wasser und dann ggf. die nicht umgesetzte Essigsäure vor oder nach der meist vorzunehmenden Hydrierung (beispielsweise nach den Angaben der DE-OS 25 37 890 oder 25 13 998) der gebildeten Butendiolderivate. Die Buten- bzw. butandiolester werden abdestilliert und das verbleibende Lösungsmittel zurückgeführt.

Die der Erfindung zugrundeliegende Reaktion wird in an sich bekannter Weise an einem festen Katalysator durchgeführt, der ein Platinmetall (Ruthenium, Rhodium, Palladium, Osmium, Iridium, Platin, insbesondere Palladium und/oder Platin) und mindestens ein Element der 5. oder 6. Hauptgruppe, vorzugsweise Antimon, Wismut, Schwefel, Selen und Tellur, insbesondere Tellur und Antimon, aber auch Eisenmetalle wie Eisen, Nikkel, Kobalt oder Kupfer enthält. Der Katalysator ist bevorzugt ein Trägerkatalysator mit Kohlenstoff als Träger. Nach der Katalysatoranordnung kann z.B. Festbett-, Suspensions- oder Wirbelbettbetrieb gewählt werden, wobei die Reaktion absatzweise oder vorzugsweise fortlaufend durchgeführt werden kann. Die Reaktionstemperatur liegt im allgemeinen zwischen 60 und 125 °C, vorzugsweise zwischen 80 und 100 °C, Temperaturen unter 60 °C sind zwar prinzipiell möglich, jedoch sinkt der Umsatz hierbei ab. Über 120 °C nimmt die Nebenproduktbildung zu. Der Reaktionsdruck ist durch die jeweils gewählte Verfahrensanordnung gegeben und liegt im allgemeinen zwischen atmosphärischem Druck und 100 bar.

Nach dem erfindungsgemässen Verfahren enstehen zunächst Butendioldiacetate (BEDA). Als Nebenprodukte konnten gaschromatographisch in Abhängigkeit vom jeweils vorhandenen Wassergehalt gewisse Anteile Butendiolmonoacetate bzw. Butendiole, die in der Regel ebenfalls Wertprodukte darstellen, und nur geringe Mengen nicht gewinnbarer bzw. verwertbarer Produkte nachgewiesen werden. Die ungesättigten Diacetate können in bekannter Weise weiterverarbeitet werden.

Bei dem Verfahren können anstelle von reinem Butadien auch butadienhaltige Kohlenwasserstoffgemische eingesetzt werden. Besonders geeignete Kohlenwasserstoffgemische sind Anteile von $C_4$-Kohlenwasserstoffen, wie sie als sog. $C_4$-Crackschnitte gewonnen werden, da das darin enthaltene Butadien erheblich kostengünstiger ist als z.B. reines Butadien.

Ein solcher $C_4$-Crackschnitt, wie er bei der Spaltung von Leichtbenzin anfällt, kann neben 1.3-Butadien ungesättigte und gesättigte Kohlenwasserstoffe, wie z.B. cis-2-Buten, trans-2-Buten, 1-Buten, iso-Buten, iso-Butan, n-Butan usw. enthalten, sollte aber, um eine Verringerung der Reaktionsgeschwindigkeit und der Katalysatorstandzeit zu vermeiden, insgesamt nicht über 0,5 Vol.- %, acetylenisch ungesättigte Kohlenwasserstoffe enthalten.

Die nach den Verfahren der Erfindung herstellbaren Butendiolester, speziell Butendioldiacetate sind wertvolle Zwischenprodukte z.B. für die Herstellung von Butandiol, Tetrahydrofuran, Adipodinitril bzw. Hexamethylendiamin und Vitamin A.

Das Verfahrensschema (Figur) zeigt den Gesamtprozess entsprechend vorliegender Erfindung.

In der ersten Stufe (A) wird Butadien (1) mit Essigsäure (2) und Sauerstoff (3) in Gegenwart geeigneter Katalysatoren zu den Butendioldiacetaten umgesetzt; Butadien und Essigsäure werden hierbei – bezogen auf Sauerstoff – im stöchiometrischen Überschuss angewandt. Diese beiden Stoffe werden in den beiden folgenden Verfahrensschritten (B, C) aus dem Reaktionsgemisch entfernt und wieder in die erste Verfahrensstufe zurückgeführt (4, 5). Bei der Butadienabtrennung wird gleichzeitig das durch Nebenreaktionen in geringer Menge entstandene $CO_2$ aus dem Kreislauf entfernt; aus der zu recyclisierenden Essigsäure muss das bei der Acetoxilierung stöchiometrisch anfallende Wasser vor dem Wiedereinsetzen in Stufe 1 abgetrennt werden (D). In einer vierten Stufe (E) werden, wenn nicht die Buten-, sondern die Butandiolderivate als Endprodukte verlangt werden, die ungesättigten Ester katalytisch hydriert. In der letzten Verfahrensstufe (F) werden die Butendiol- bzw. Butandiolester aus dem höhersiedenden Lösungsmittel (6) destillativ abgetrennt; sie können – wenn erforderlich – durch Rektifikation gereinigt und/oder in die einzelnen Verbindungen aufgetrennt werden.

Beispiel 1
Versuch 1 (Vergleichsversuch)
In einen Reaktionsbehälter mit Begasungsrührer werden 15 g Katalysator (6,4% Pd, 1,5% Te auf Aktivkohle, 0,1 – 0,2 mm Körnung, hergestellt analog Beispiel 33 der DE-OS 22 17 452), 600 g Essigsäure und zunächst kein Verdünnungsmittel gegeben. Bei 95 °C wird ein Gemisch aus 3 Nl/h Sauerstoff und 3 Nl/h Butadien eingeleitet. Nach einer Reaktionszeit von 4 Stunden bei den angegebenen Bedingungen wird die Reaktion abgebrochen, der Katalysator abgetrennt und das dabei erhaltene Rohfiltrat gaschromatographisch untersucht bzw. destillativ (Kolonne) aufgearbeitet. Es werden 30,7 g Butendioldiacetate gefunden (ungefähre Zusammensetzung: 70% trans-1,4-BEDA, 20% cis-1,4-BEDA, 10% 3,4-BEDA). Butendiolmonoacetate und Butendiole werden nur in Spuren nachgewiesen.

Versuch 2 bis 8

Es wird verfahren wie im Versuch 1 beschrieben, wobei unter Beibehaltung aller sonstigen Bedingungen die Essigsäure stufenweise durch Sulfolan ersetzt wird.

In der Tabelle sind die Ergebnisse der Versuchsreihe zusammengestellt. In der letzten Spalte ist die «Katalysatorproduktivität» des jeweiligen Versuchs in g BEDA pro kg Katalysator und Stunde angegeben. Die Verteilung der einzelnen BEDA-Isomeren entspricht der in Versuch 1 angegebenen. In Abbildung 2 ist die BEDA-Bildung bei diesen Versuchen in Abhängigkeit von der Essigsäure- bzw. Sulfolan-Menge graphisch aufgetragen.

Tabelle:

| Versuch Nr. | Essigsäure (g) | Sulfolan (g) | BEDA (g) | RZA g BEDA / kg Kat.h |
|---|---|---|---|---|
| 1 | 600 | 0 | 30,7 | 511 |
| 2 | 550 | 50 | 25,4 | 423 |
| 3 | 500 | 100 | 22,7 | 378 |
| 4 | 450 | 150 | 21,6 | 360 |
| 5 | 400 | 200 | 20,5 | 342 |
| 6 | 300 | 300 | 16,5 | 275 |
| 7 | 180 | 420 | 12,1 | 202 |
| 8 | 90 | 510 | 7,5 | 125 |

Vergleichsbeispiel zu Beispiel 1

Es wird verfahren wie im Beispiel 1 beschrieben, wobei unter Beibehaltung aller sonstigen Bedingungen nur 300 g Essigsäure (ohne Sulfolan) eingesetzt werden. Man erhält 23,4 g BEDA-Fraktion, vergleichbar zusammengesetzt wie im Versuch 1.

Beispiel 2

Es wird verfahren wie im Beispiel 1, Versuch 6, beschrieben, wobei unter Beibehaltung der sonstigen Bedingungen anstelle von 300 g Sulfolan 300 g Siliconöl (Phenylmethylpolysiloxan AP 100 der Firma Wacher-Chemie) eingesetzt werden (15 g Katalysator mit 7,1% Pd und 1,2% Te auf Aktivkohle, hergestellt wie im Beispiel 1 angegeben). Nach Abtrennung von Wasser und noch vorhandener Essigsäure erhält man 42,1 g BEDA (Summe der Isomeren).

Vergleichsbeispiel zu Beispiel 2

Es wird verfahren wie in Beispiel 2 beschrieben, wobei – unter sonst gleichen Versuchsbedingungen – als Lösungsmittel nur 600 g Essigsäure (ohne Siliconöl) eingesetzt werden. Man erhält 65,3 g BEDA.

Beispiel 3

Es wird verfahren wie im Beispiel 1, Versuch 5, angegeben, wobei unter Beibehaltung der sonstigen Bedingungen 400 g Essigsäure und 200 g Dimethoxy-äthylphthalat (anstelle von Sulfolan) eingesetzt werden (Katalysator wie im Beispiel 1). Es werden 16,1 g BEDA-Isomere erhalten.

Beispiel 4

Es wird verfahren wie im Beispiel 1, Versuch 5, angegeben, wobei unter Beibehaltung der sonstigen Bedingungen 400 g Essigsäure und anstelle von Sulfolan 200 g einer auf der Grundlage eines Isomerengemisches von Triaryldimethanen aufgebauten Wärmeübertragungsflüssigkeit, die unter dem Handelsnamen Marlotherm S (Hersteller Chemische Werke Hüls) erhältlich ist, eingesetzt werden. Es werden 5 g BEDA destillativ erhalten.

Beispiel 5

Es wird verfahren wie in Beispiel 1, Versuch 6, angegeben, wobei anstelle von Essigsäure 300 g reine Propionsäure angewandt werden. Nach 6 Stunden Reaktionszeit werden nach entsprechender Aufarbeitung 24 g einer Mischung aus trans- bzw. cis-2-buten-1,4-dioldipropionat und 1-Buten-3,4-dioldipropionat erhalten.

**Patentanspruch**

Verfahren zur Herstellung von Butendioldiacetat oder Butendioldipropionat durch Umsetzung von Butadien mit Sauerstoff und Essigsäure bzw. Propionsäure oder einer Verbindung, die unter den Reaktionsbedingungen Essigsäure bzw. Propionsäure freisetzt, insbesondere Methylacetat bzw. Methylpropionat, an einem festen Katalysator, der ein Platinmetall und mindestens ein Element der 5. oder 6. Hauptgruppe enthält, dadurch gekennzeichnet, dass die Umsetzung in Gegenwart eines Lösungsmittels durchgeführt wird, das einen höheren Siedepunkt als die Butendioldiacetate bzw. Butendioldipropionate aufweist und das die Reaktion weder chemisch beeinflusst noch von ihr verändert wird.

**Revendication**

Procédé pour la préparation de diacétate de butène-diol ou de dipropionate de butène-diol par réaction de butadiène avec l'oxygène et l'acide acétique ou l'acide propionique ou avec un composé qui libère de l'acide acétique ou de l'acide propionique dans les conditions de la réaction, en particulier l'acétate de méthyle ou le propionate de méthyle, avec un catalyseur solide qui contient un métal du groupe du platine et au moins un élément du groupe principal V ou VI, caractérisé en ce que la réaction est menée en présence d'un solvant qui présente un point d'ébullition plus élevé que les diacétates de butène-diol ou les dipropionates de butène-diol et qui n'influe pas chimiquement sur la réaction, ni n'est modifié par celle-ci.

**Claim**

A process for the preparation of butenediol diacetate or butenediol dipropionate by reacting butadiene with oxygen and acetic acid or propionic acid, or a compound which liberates acetic acid or propionic acid under the reaction conditions, especially methyl acetate or methyl propionate, over a solid catalyst which contains a platinum metal and at least one element of main group 5 or 6, characterized in that the reaction is carried out in the presence of a solvent which has a higher boiling point than the butenediol diacetates or butenediol dipropionates, and which neither chemically influences the reaction nor is altered by it.

1/1